# FASCICULE DE BREVET EUROPEEN

(11) **EP 0 477 178 B1**
(45) Date de publication et mention de la délivrance du brevet: **22.09.1993**
(21) Numéro de dépôt: 90904867.0
(22) Date de dépôt: 16.03.1990
(51) Int. Cl.: A61K 31/505, A61K 31/28

(54) **COMPOSITION THERAPEUTIQUE ET COFFRET POUR LE TRAITEMENT DE LA POLYARTHRITE RHUMATOIDE**
THERAPEUTISCHE ZUSAMMENSETZUNG UND KIT FÜR DIE BEHANDLUNG DER RHEUMATISCHEN POLYARTHRITIS
THERAPEUTIC COMPOSITION AND KIT FOR THE TREATMENT OF RHUMATOID POLYARTHRITIS

(30) Priorité: 17.03.1989 FR 8903553
(43) Date de publication de la demande: 01.04.1992
(73) Titulaire: LABORATOIRES DE THERAPEUTIQUE MODERNE, 92151 Suresnes Cédex (FR)
(72) Inventeur: BITTER, Thomas, CH-1003 Lausanne (CH); WIERZBICKI, Norbert, F-94300 Vincennes (FR); BELORGEY-BISMUT, Chantal, F-75014 Paris (FR); CHRISTEN, Marie-Odile, F-75016 Paris (FR)
(74) Mandataire: Le Guen, Gérard
(86) Numéro de dépôt international: FR9000180
(87) Numéro de publication internationale: WO9011076

(56) Documents cités:
- US-A- 4 657 763
- Chemical Abstracts, vol. 106, no. 7, 16 Febr. 1987, (Columbus, Ohio, US) S.C. Ridge et al., p. 37, abstract no. 43652p
- Chemical Abstracts, vol. 75, no. 11, 13 Sept. 1971, (Columbus, Ohio, US) D.T. Walz et al., p. 295, abstract no. 74564g

## Description

La présente invention est relative à de nouvelles compositions et présentations thérapeutiques pour le traitement de la polyarthrite rhumatoïde.

La polyarthrite rhumatoïde est une maladie inflammatoire chronique qui peut atteindre toutes les articulations.

Son évolution spontanée se fait par poussées inflammatoires; les malades présentent des oedèmes des articulations et des déformations articulaires. Ces atteintes peuvent conduire à une impotence.

Sur le plan biologique, on peut noter des signes de l'inflammation.

La présente invention est basée sur l'utilisation de deux produits déjà connus dans cette indication. Il s'agit de l'aurothiopropanolsulfonate de sodium et du méthotrexate.

L'aurothiopropanolsulfonate de sodium est utilisé depuis plus d'un demi-siècle pour le traitement de la polyarthrite rhumatoïde. Il existe d'autres sels d'or sur le marché.

L'aurothiopropanolsulfonate de sodium est commercialisé sous forme injectable (Allochrysine®). Les injections sont hebdomadaires puis mensuelles. Les doses sont augmentées progressivement en partant de 2,5 cg. Les injections deviennent mensuelles et sont poursuivies soit jusqu'à épuisement de l'effet thérapeutique, soit jusqu'à l'apparition d'une intolérance aux sels d'or.

Le méthotrexate est également utilisé depuis les années 50 pour le traitement de la polyarthrite rhumatoïde. Il s'agit d'un antagoniste de l'acide folique. Il est utilisé dans les polyarthrites rebelles ou intolérantes aux traitements habituellement utilisés.

Les doses utilisées sont habituellement injectées par voie intra-musculaire et sont d'environ 7,5 à 50 mg par semaine ou bien administrées par voie orale dans les mêmes fourchettes de concentration pendant des durées décrites dans la littérature allant jusqu'à 48 mois.

La toxicité du méthotrexate utilisé à ces doses est relativement faible. Les effets secondaires les plus préoccupants du méthotrexate sont les atteintes pulmonaires ou hépatiques dont l'incidence reste à évaluer dans cette indication.

La présente invention est basée sur la découverte que l'association de l'aurothiopropanolsulfonate de sodium et de méthotrexate permet de traiter les malades devenus résistants aux sels d'or et/ou d'augmenter la dose totale injectée sans faire apparaître d'effets toxiques.

La présente invention a ainsi pour objet une composition thérapeutique pour le traitement de la polyarthrite rhumatoïde comprenant en association de l'aurothiopropanolsulfonate de sodium et du méthotrexate.

La présente invention a également pour objet un coffret pour le traitement de la polyarthrite rhumatoïde comprenant de façon séparée une composition contenant de l'aurothiopropanolsulfonate de sodium et une composition contenant du méthotrexate.

Dans la présente invention les doses utilisées pour l'aurothiopropanolsulfonate de sodium (ALLOCHRYSINE®) sont généralement de 25 à 200 mg par semaine.

La dose de méthotrexate utilisée est généralement de 5 à 50 mg par semaine.

Le coffret contient soit une ampoule dosée de 25 à 100 mg et préférentiellement à 100 mg d'aurothiopropanol sulfonate de sodium en solution et une ampoule dosée de 5 à 50 mg et préférentiellement à 10 mg de méthotrexate en solution; soit une ampoule dosée de 25 à 100 mg et préférentiellement à 100 mg d'aurothiopropanol sulfonate de sodium en solution, un flacon de lyophilisat de 5 à 50 mg et préférentiellement de 20 mg de methotrexate et une ampoule d'eau pour préparations injectables.

Dans les deux cas, le mélange des deux produits est effectué extemporanément avant l'injection.

L'administration peut être effectuée notamment par voie intra-musculaire ou intra-veineuse. Dans le cas d'une injection intra-musculaire, l'ALLOCHRYSINE® sans diluant peut être directement mélangée au méthotrexate.

Il n'a pas été détecté d'intolérance locale au site d'injection.

## Revendications

1. Composition thérapeutique injectable pour le traitement de la polyarthrite rhumatoïde comprenant en association de l'aurothiopropanol-sulfonate de sodium et du méthotrexate.

2. Coffret pour le traitement de la polyarthrite rhumatoïde comprenant de façon séparée une composition injectable contenant de l'aurothiopropanolsulfonate de sodium et une composition injectable contenant du méthotrexate.

## Claims

1. Injectable therapeutic composition for the treatment of rheumatoid polyarthritis comprising, in combination, sodium aurothiopropanolsulphonate and methotrexate.

2. Kit for the treatment of rheumatoid polyarthritis comprising separately an injectable composition containing sodium aurothiopropanolsulphonate and an injectable composition containing methotrexate.

## Patentansprüche

1. Injizierbare therapeutische Zusammensetzung für die Behandlung der rheumatischen Polyarthritis, die in Assoziation Natriumaurothiopropanolsulfat und Methotrexat umfaßt.

2. Kit für die Behandlung der rheumatischen Polyarthritis, welches getrennt eine Natriumaurothiopropanolsulfonat beinhaltende, injizierbare Zusammensetzung und eine Methotrexat beinhaltende, injizierbare Zusammensetzung umfaßt.
